(19) Europäisches Patentamt
European Patent Office
Office européen des brevets

(11) Publication number: 0 372 571 A2

(12) **EUROPEAN PATENT APPLICATION**

(21) Application number: 89122689.6

(22) Date of filing: 08.12.89

(51) Int. Cl.5: C07C 69/18, C07C 43/15, C07C 309/66, C07C 247/08, C07C 233/18, C07C 217/46, C07H 15/10

(30) Priority: 09.12.88 JP 311260/88

(43) Date of publication of application: 13.06.90 Bulletin 90/24

(84) Designated Contracting States: AT BE CH DE ES FR GB GR IT LI LU NL SE

(71) Applicant: MECT CORPORATION
2-1-1, Nishi-Shinjyuku Shinjyuku-ku
Tokyo(JP)

(72) Inventor: Fujita, Shuji
2-17-3, Kashiwa-cho
Tachikawa-shi Tokyo(JP)

Inventor: Sugimoto, Mamoru
1-26-8, Kamitakada
Nakano-ku Tokyo(JP)
Inventor: Ito, Masayoshi
1-27-22-303, Fujimidai
Kunitachi-shi Tokyo(JP)
Inventor: Ogawa, Tomoya
3-6-6-101, Kichijyojikitamachi
Musashino-shi Tokyo(JP)

(74) Representative: Vossius & Partner
Siebertstrasse 4 P.O. Box 86 07 67
D-8000 München 86(DE)

# (54) Novel sphingenine and derivatives thereof.

(57) A sphingenine and derivatives thereof herein provided are represented by the following general formulas (I) and (II):

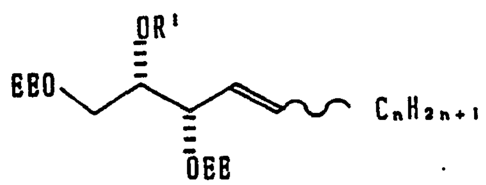

(I)

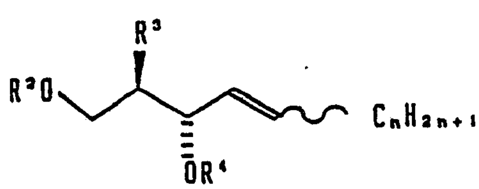

(II)

in the formulas (I) and (II), $R^1$ represents a hydrogen atom, an acetyl group or a methanesulfonyl group; $R^2$ represents a hydrogen atom or an ethoxyethyl group; $R^3$ represents an azido group, an amino group, an

EP 0 372 571 A2

alkylamido group, an arylamido group, an aralkylamido group or a tetracosanamido group; $R^4$ represents a hydrogen atom or an ethoxyethyl group; EE represents an ethoxy ethyl group; and n is an integer ranging from 1 to 22. These sphingenine and derivatives thereof are very useful as intermediates for synthesizing glycolipids and gangliosides which are effective as markers of tumours, in particular, in the preparation of ceramide parts of the glycolipids and gangliosides.

## Novel Sphingenine and Derivatives Thereof

The present invention relates to a novel sphingenine and derivatives thereof and more specifically to a sphingenine useful as an intermediate for the synthesis of glycolipids and gangliosides, as well as derivatives thereof.

Glycolipids of mammalian cells belong to so-called sphingoglycolipids and comprise (i) a lipid structure composed of a long chain aminoalcohol called ceramide to which a fatty acid is bonded through an amido bond and (ii) a combination of sugars selected from the group consisting of, for instance, glucose, galactose, N-acetylglucosamine, N-acetylgalactosamine, fucose and sialic acid, which is bonded to the lipid structure through a glycoside bond. Among them, sialic acid-containing glycosphingolipids are called gangliosides.

Most of these compounds are in general localized in external side of the plasma membranes and it is thought from the recent studies that they play an important role in various biological functions such as recognitions, differentiations and receptors of cells.

However, it is quite difficult to isolate and purify the sphingoglycolipids from various animal cells and tissues. Therefore, exact synthesis of such ceramide compounds which constitute the sphingoglycolipids is indispensable for elucidating the correlation between the correct biological information of these glycolipids and their molecular structures.

There has been developed a method for stereo-selectively synthesizing their natural ceramide portions among others in a good yield (see JP-A-60-190745).

Moreover, the natural sphingosines are optically active substances whose erythro configuration is in D-series (2S, 3R) and there have been a large number of methods for synthesizing them from D-glucose, D-galactose and L-serine as starting materials.

In addition, as to the synthetic and semi-synthetic ceramides, a method for synthesizing trans-DL-threo isomer and cis-DL-threo isomer from an achiral compound as a starting material has been proposed by Grob et al., (see Grob C.A. et al., Helv. Chem. Acta, 1957, 40, p. 1145).

To obtain optically active substances without optical resolution, it is necessary to use optically active substances as starting materials. However, most of such optically active substances are very expensive and their mass-production is in general very difficult.

Accordingly, an object of the present invention is to provide a novel sphingenine which is an optically active compound having an erythro configuration (2R, 3S) and, which may be used as a key intermediate for synthesizing gangliosides.

Another object of the present invention is to provide derivatives of such a novel optically active sphingenine.

A further object of the present invention is to provide a method for preparing such a novel sphingenine and derivatives thereof.

These objects were achieved on the basis of the surprising finding that a novel optically active synthetic or semi-synthetic ceramide can be synthesized and isolated by using a less expensive and pure chiral compound as a starting material, without requiring any optical resolution processes.

According to an aspect of the present invention, there is provided a novel sphingenine and derivatives thereof represened by the following general formula (I) or (II):

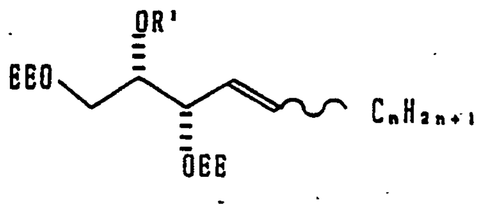

(I)

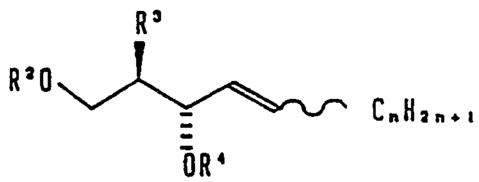

(II)

In the formulas (I) and (II), $R^1$ represents a hydrogen atom, an acetyl group or a methanesulfonyl group; $R^2$ represents a hydrogen atom or an ethoxyethyl group; $R^3$ represents an azido group, an amino group, an alkylamido group, an arylamido group, an aralkylamido group or a tetracosanamido group; $R^4$ represents a hydrogen atom or an ethoxyethyl group; EE represents an ethoxy ethyl group; and n is an integer ranging from 1 to 22.

The present invention will hereinafter be explained in more detail.

First of all, the method for preparing the compounds of the present invention will hereunder be explained in more detail with reference to the following reaction schemes (I) and (II).

Reaction Scheme (I)

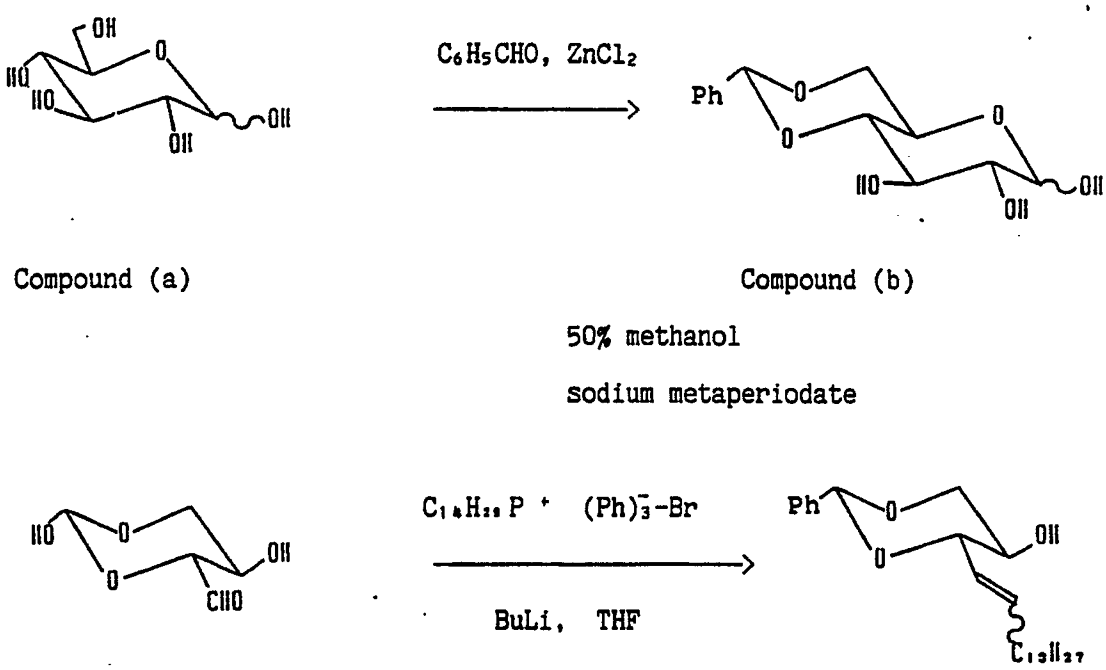


Compound (a)

Compound (b)

Compound (c)

(Ph: phenyl group)

Compound (d)

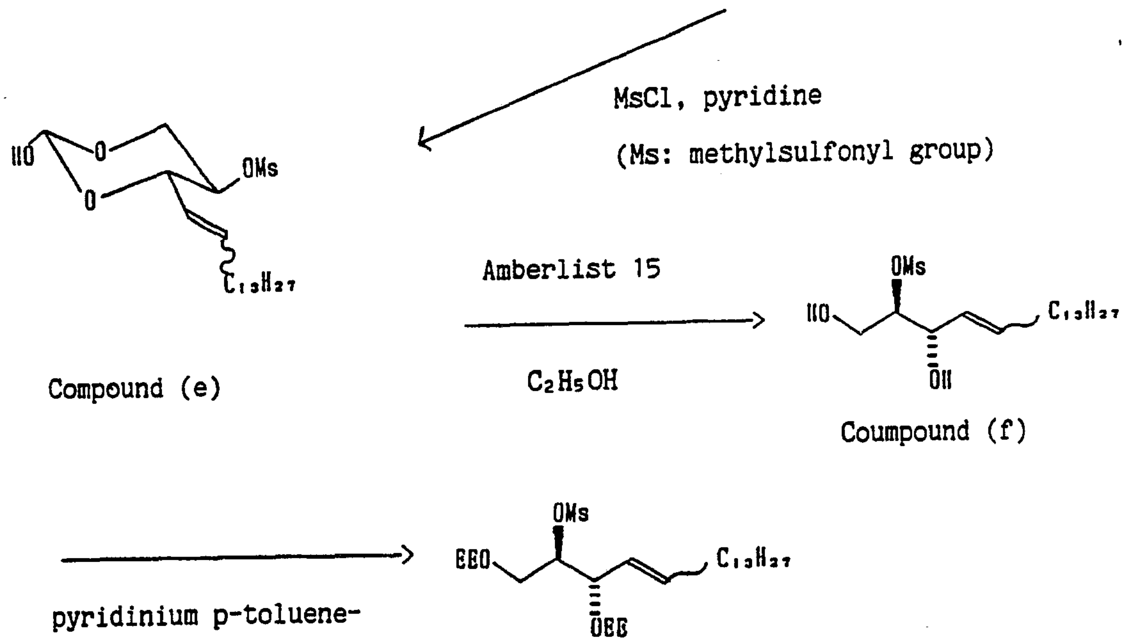


Compound (e)

Coumpound (f)

Compond (1)

(EE: ethoxyethyl group)

Reaction Scheme (II)

(1)

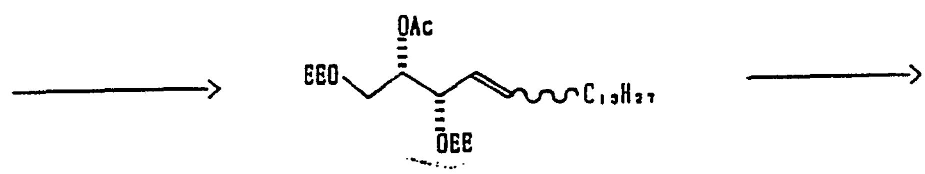

Compound (2)

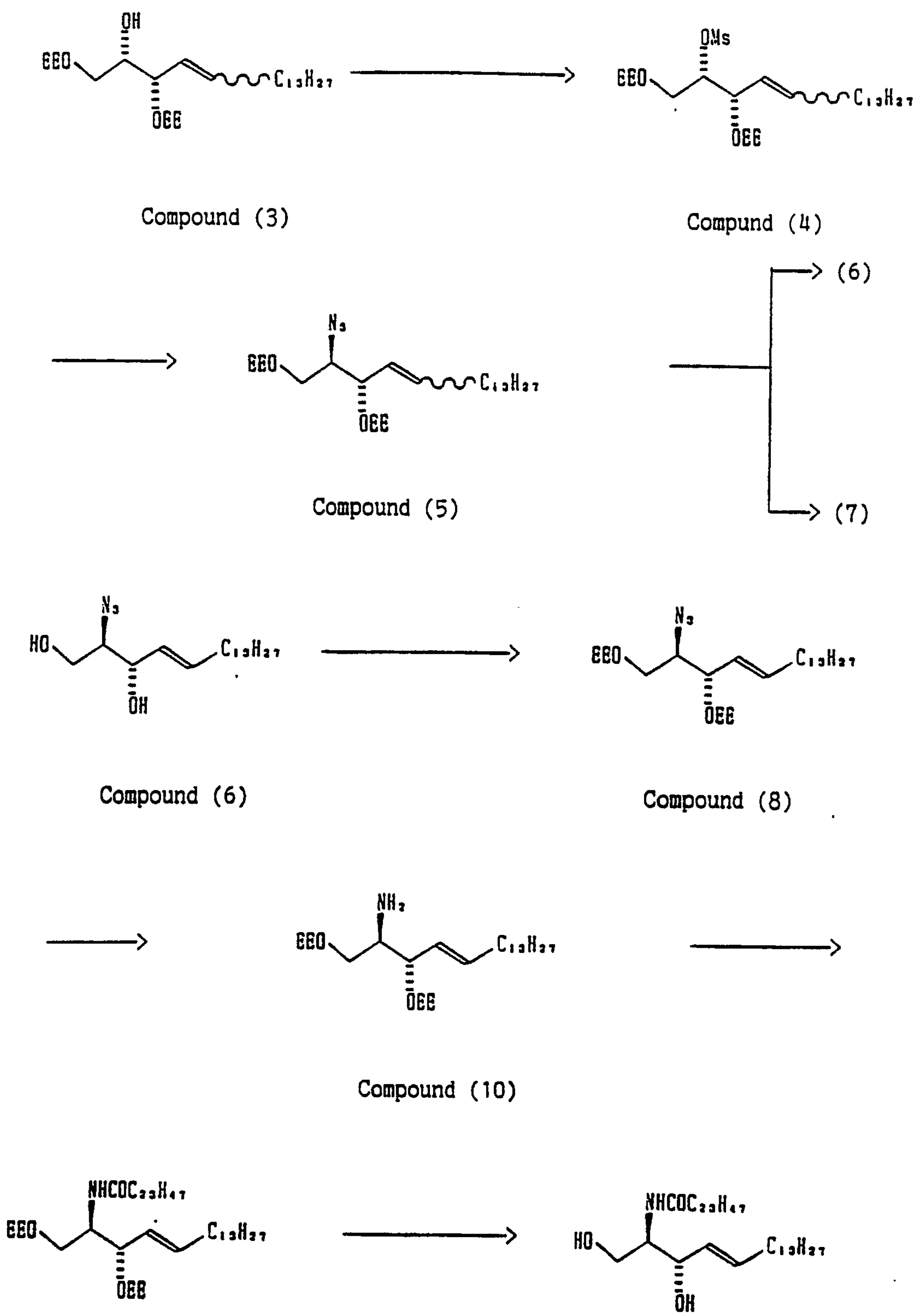

Compound (3)

Compund (4)

Compound (5)

Compound (6)

Compound (8)

Compound (10)

Compound (12)

Compound (14)

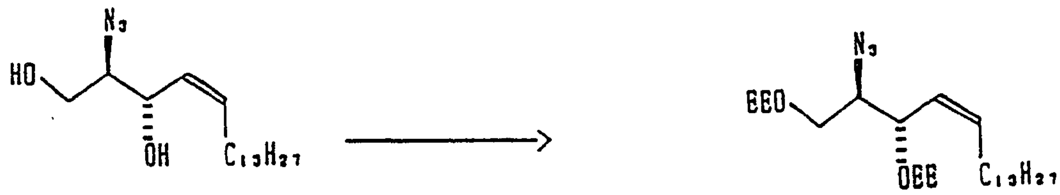

Compound (7) Compound (9)

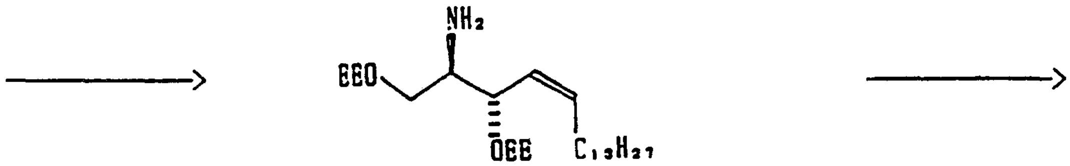

Compound (11)

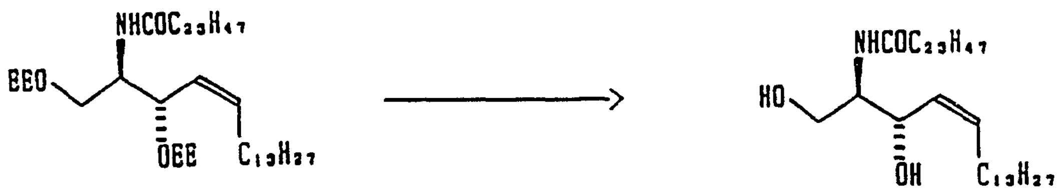

Compound (13) Compound (15)

(1) Preparation of the Compound (b)

A known compound (a) is reacted under the following reaction conditions to form the compound (b):

A catalyst such as $ZnCl_2$ may be used in this reaction. In addition, benzaldehyde as a reactant may also serve as a reaction solvent. Moreover, the reaction can be performed by stirring the reaction mixture for about 6 to 36 hours, in particular 12 hours. The reaction can be carried out at a temperature ranging from about 15 to 50 ° C and preferably at room temperature. The reaction product thus obtained may be purified by an ordinary manner such as recrystallization.

(2) Preparation of the Compound (c)

The compound (c) can be prepared by reacting the foregoing compound (b) under the following reaction conditions:

A catalyst such as sodium metaperiodate may be used in this reaction. Methanol may be used in this reaction as a solvent. Preferably, 50% methanol is used as the reaction solvent. The reaction can be carried out by stirring the reaction mixture for about 1 to 8 hours, in particular is 4 hours. The reaction can be carried out at a temperature ranging from about 10 to 50 °C and preferably at room temperature.

(3) Preparation of the Compound (d)

The compound (d) can be prepared by reacting the foregoing compound (c) with $C_{14}H_{29}P^{+}(Ph)_3 \cdot Br^{-}$ under the following reaction conditions:

As a catalyst for this reaction, there may be used butyl lithium or phenyl lithium, preferably butyl lithium. Examples of reaction solvents are tetrahydrofuran (THF) and hexane, preferably THF. This reaction can be performed by stirring the reaction mixture for about 0.5 to 20 hours, preferably 20 hours. In addition, the reaction may be carried out at a temperature ranging from about -30 to -15 °C and preferably -20 °C. The reaction product thus obtained can be purified in an ordinary manner such as a column chromatography technique.

(4) Preparation of the Compound (e)

The compound (e) can be prepared by subjecting the foregoing compound (d) to methylsulfonylation under the following reaction conditions:

As a reaction solvent, pyridine may be used. This reaction can be performed by stirring the reaction mixture for about 2 to 30 hours, preferably 20 hours. In this case, the reaction temperature ranges from about 0 to 25 °C and preferably 20 °C. The reaction product thus obtained may be purified in an ordinary manner such as a column chromatography technique.

(5) Preparation of the Compound (f)

The compound (f) can be obtained by removing acetal groups from the foregoing compound (e) under the following reaction conditions:

As a catalyst, there may be used, for instance, hydrochloric acid, p-toluenesulfonic acid or Amberlist 15 and preferably Amberlist 15. As a reaction solvent, there may be used, for instance, methanol or ethanol, preferably ethanol. The reaction may be performed by stirring the reaction mixture for about 5 to 15 hours, preferably 8 hours. Moreover, the reaction temperature of this reaction ranges from about 60 to 80 °C and preferably 65 °C. The reaction product thus obtained may be purified in an ordinary manner such as a column chromatography technique.

(6) Preparation of the Compound (1)

The compound (1) can be obtained by reacting the foregoing compound (f) with ethyl vinyl ether ($EtOCH=CH_2$) under the following reaction conditions:

In this reaction, there may be used, for instance, pyridinium p-toluenesulfonate as a reaction catalyst. Examples of the reaction solvents are dichloromethane, dichloroethane and chloroform. Preferred reaction solvent is dichloromethane. This reaction can be carried out by stirring the reaction mixture for about 0.5 to 20 hours, preferably one hour. Moreover, the reaction temperature of this reaction ranges from about 0 to 30 °C and preferably 20 °C. The reaction product thus obtained may be purified in an ordinary manner such as a column chromatography technique.

(7) Preparation of the Compound (2)

The compound (2) may be obtained by acetylating the foregoing compound (1) under the following reaction conditions:

As agents for acetylation, there may be mentioned, for instance, CsOAc, NaOAc and KOAc. Particularly preferred is CsOAc among others. It is desirable in this reaction that the agent for acetylation be used in an amount of about 1 to 20 equimolar, preferably about 15 to 18 equimolar the molar amount of the compound (1). As a reaction solvent, there may be used, for instance, dimethylformamide (DMF), THF and $CH_2Cl_2$ and preferably DMF. The reaction is desirably performed at a temperature ranging from about 40 to 150° C, preferably about 100 to 120° C for 3 to 200 hours, preferably about 100 to 150 hours. The reaction product thus obtained may be purified in an ordinary manner such as a column chromatography technique.

(8) Preparation of the Compound (3)

The compound (3) can be prepared by deacetylating the foregoing compound (2) under the following reaction conditions:

As the deacetylating agents, there may be used, for instance, an alkoxide such as NaOMe, and an alkali such as NaOH and KOH. Particularly preferred deacetylating agent is NaOMe. The deacetylating agent may be used in an amount of about 0.1 to 2 equimolar the molar amount of the compound (2) and preferably an amount approximately equal to that of the compound (2). As a reaction solvent, there may be used, for instance, a mixed solvent of methanol and dichloromethane; ethanol, propanol; and a mixed solvent of ethanol or propanol with chloroform, THF or benzene and in particular it is preferred to use a mixed solvent of methanol and dichloromethane. The reaction is desirably performed at a temperature ranging from about 0 to 60° C, preferably about 20 to 30° C for 30 minutes to 24 hours, preferably about 3 to 5 hours. The reaction product thus obtained may be purified in an ordinary manner such as a column chromatography technique.

(9) Preparation of the Compound (4)

The compound (4) can be prepared by subjecting the foregoing compound (3) to methylsulfonylation under the following reaction conditions:

As a methylsulfonylation agent, there may be used, for instance, MsCl, $Ms_2O$ or MsBr (wherein Ms represents a methylsulfonyl group) and in particular MsCl is preferably used in this reaction. The methylsulfonylation agent may be used in the reaction in an amount of about 1 to 5 equimolar, preferably about 1.5 to 2 equimolar the molar amount of the compound (3). Examples of the reaction solvents are pyridine, dichloromethane, chloroform and benzene, in particular pyridine. The reaction is desirably carried out at a temperature ranging from about 0 to 60 ° C, preferably about 20 to 30 ° C for about 1 to 48 hours, preferably about 15 to 25 hours in the presence of a reaction catalyst such as pyridine, dimethylaminopyridine or a tertiary amine such as triethylamine. The reaction product thus obtained may be purified in an ordinary manner such as a column chromatography technique.

(10) Preparation of the Compound (5)

The compound (5) can be prepared by converting the foregoing compound (4) into an azide under the following reaction conditions:

As an agent for forming azides, there may be mentioned, for instance, $NaN_3$, $(n\text{-}Bu)_4NN_3$ (tetra-n-butyl ammonium azide) and $TMSN_3$ (trimethylsilyl azide). In particular, $NaN_3$ is preferably used in this reaction. The agent for forming azides may be used in an amount of about 1 to 10 equimolar, preferably about 5 to 7 equimolar the molar amount of the foregoing compound (4). Solvents such as DMF and THF may be used in the reaction. The reaction is desirably carried out at a temperature ranging from about 60 to 150° C preferably about 100 ° C for about 3 to 100 hours, preferably about 40 to 50 hours. The reaction product thus obtained may be purified in an ordinary manner such as a column chromatography technique.

(II) Preparation of the Compounds (6) and (7)

These compounds (6) and (7) can be prepared by removing ethoxyethyl group from the foregoing compound (5) under the following reaction conditions:

As an agent for removing ethoxyethyl group, there may be used, for instance, trifluoroacetic acid

($CF_3COOH$), acetic acid, an acidic ion-exchange resin such as Amberlist 15, HCl, $H_2SO_4$ and TsOH (p-toluenesulfonic acid). In particular, the use of $CF_3COOH$ is preferred in this reaction. The agent for removing ethoxyethyl groups is desirably used in the reaction in an amount ranging from about 2 to 10 equimolar, preferably about 2 to 3 equimolar the molar amount of the foregoing compound (5). A reaction solvent such as a mixed solvent of methanol with dichloromethane, ethanol, propanol, or a mixed solvent of ethanol or propanol with chloroform, THF or benzene, in particular a mixed solvent of methanol with dichloromethane is preferably used in the reaction. The reaction is desirably carried out at a temperature ranging from about 0 to 60° C, preferably about 20 to 30° C for about 1 to 24 hours, preferably about 5 to 8 hours. The resultant reaction product contains the compounds (6) and (7). Thus, the reaction product is first subjected to fractional recrystallization from a solvent such as hexane to separate a part of the compound (6) as crystals and then the resulting mother liquor is treated with column chromatography to separate the remaining compound (6) from the compound (7). The column chromatography is preferably carried out utilizing a silica gel column and a mixed solvent of hexane with ethyl acetate as an eluent.

(12) Preparation of the Compounds (8) and (9)

The foregoing compounds (6) and (7) each is subjected to ethoxyethylation under the following reaction conditions to obtain the corresponding compounds (8) and (9).

It is preferred to use ethyl vinyl ether ($EtOCH=CH_2$) as an agent for ethoxyethylation in this reaction. The agent for ethoxyethylation is suitably used in the reaction in an amount ranging from about 3 to 20 equimolar, preferably about 10 equimolar the molar amount of the corresponding compound (8) or (9). Examples of solvents used in this reaction include dichloromethane, chloroform, 1,2-dichloroethane and benzene and preferably dichloromethane. The reaction is desirably carried out at a temperature ranging from about 0 to 60° C, preferably about 20 to 30° C for about 0.5 to 6 hours, preferably about 1 to 2 hours, in tie presence of a catalyst such as pyridinium p-toluenesulfonate (PPTS). The reaction product thus obtained may be purified in an ordinary manner such as a column chromatography technique.

(13) Preparation of the Compounds (10) and (11)

The foregoing compounds (8) and (9) each is reduced under the following reaction conditions to form the corresponding compound (10) or (11).

The reduction can be performed using $NaBH_4$ as a reducing agent or a combination of $H_2$ gas with a Lindlar catalyst or a combination of $H_2S$ gas with pyridine. In particulars, the use of $NaBH_4$ is preferred in this reaction. The reduction is preferably carried out in a reaction solvent such as an alcohol, e.g., isopropanol, methanol. ethanol, n-propanol or butanol, in the presence of a reducing agent in an amount ranging from about 2 to 20 equimolar, preferably about 6 to 10 equimolar amount of the compound (8) or (9). Alternatively, when $H_2$ gas is used, the reduction is performed under a hydrogen gas pressure ranging from about 1 . 1 to 4 atm. while if $H_2S$ gas is employed, its pressure is preferably about one atm. The reduction is desirably carried out at a temperature ranging from about 20 to 100 ° C, preferably at a reflux temperature for about 8 to 60 hours, preferably about 40 to 45 hours. The reaction product thus obtained may be purified in an ordinary manner such as a column chromatography technique.

(14) Preparation of the Compounds (12) and (13)

The compound (12) or (13) can be prepared by reacting the foregoing compound (10) or (11) with lignoceric acid ($C_{23}H_{47}$ COOH) under the following reaction conditions:

Examples of the reaction solvents used in the reaction are dichloromethane, chloroform and dichloroethane and preferably dichloromethane. The reaction is suitably carried out at a temperature ranging from about 20 to 100° C, preferably a reflux temperature for about 0.5 to 10 hours, preferably about 1 to 2 hours in the presence of a catalyst such as 2-chloro-1-methylpyridinium iodide represented by the following formula:

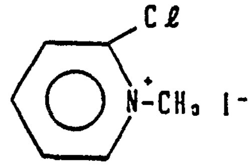

or dicyclohexyl carbodiimide (DCC) or diphenyl phosphoryl azide (DPPA). Lignoceric acid is desirably used in this reaction in an amount ranging from about 1 to 3 equimolar, preferably about 1.1 to 1.3 equimolar the molar amount of the compound (10) or (11). The reaction product thus obtained may be purified in an ordinary manner such as a column chromatography technique.

(15) Preparation of the Compounds (14) and (15)

These compounds (14) and (15) each can be prepared by removing ethoxyethyl group from the foregoing compound (12) or (13) under the following reaction conditions:

As an agent for removing ethoxyethyl group, there may be used, for instance, $CF_3COOH$, acetic acid, an acidic ion-exchange resin such as Amberlist-15, HCl, $H_2SO_4$ and TsOH (p-toluenesulfonic acid). In particular, the use of $CF_3COOH$ is prefered in this reaction. The agent for removing ethoxyethyl groups is desirably used in the reaction in an amount ranging from about 2 to 10 equimolar, preferably about 2 to 3 equimolar amount of the foregoing compound (12) or (13). A reaction solvent such as a mixed solvent of methanol with dichloromethane, ethanol, propanol, or a mixed solvent of ethanol or propanol with chloroform, THF or benzene, in particular a mixed solvent of methanol with dichloromethane is preferably used in the reaction. The reaction is desirably carried out at a temperature ranging from about 0 to 60 ° C, preferably about 20 to 30° C for about 1 to 24 hours, preferably about 5 to 8 hours. The reaction product thus obtained may be purified in an ordinary manner such as a column chromatography technique.

All of the compounds (2) to (15) according to the present invention are novel compounds.

The novel sphingenine and derivatives thereof according to the present invention can be effectively used as an intermediate for synthesizing glycolipids and gangliosides which would be useful as markers for tumours, more specifically as an intermediate for synthesizing the ceramide part in preparing the glycolipids and the gangliosides.

The present invention will hereunder be explained in more detail with reference to the following working Examples and Reference Examples, but the present invention is not restricted to these specific Examples given below.

Reference Example 1: Preparation of Compound (b) (4, 6-O-benzylidene-D-glucose)

To the compound (a) (80 g; 0.44 moles), there were added 1 ℓ (8.5 moles) of benzaldehyde and 320 g (2.4 moles) of zinc chloride and the mixture was vigorously stirred at room temperature over night.

Then, 1.5 ℓ of water was added to the reaction solution, followed by stirring it for one hour, filtering off the resultant precipitates, washing the precipitates with n-heptane and then drying them in vacuo to obtain 19.81 g of white powder.

Moreover, the filtrate was extracted with ether, washed with a saturated common salt solution and then dried over anhydrous magnesium sulfate. The solvent was distilled off in a reduced pressure to obtain 70.74 g of a pale yellow residue. The residue was combined with the white powder obtained above and the mixture was recrystallized from ethanol to thus obtain 74.26 g (62.8%) of the title compound (b) as colorless needles.

Physicochemical Properties of the Compound (b)

TLC (silica gel/chloroform-methanol (7:1)): Rf = 0.31
m.p.: 149 - 153 ° C (Lit (See "Methods in Carbohydr. Chem., 1963, 2, p. 307.) m.p. = 155 - 161 ° C) IR $\nu_{max}$ (KBr) $cm^{-1}$ : 3582, 3316, 1452, 1387, 1367, 1094, 1008.

Reference Example 2: Preparation of Compound (c) (2,4-O-benzylidene-D-erythrose)

The compound (b) (13.4 g; 0.05 mole) was dissolved in 150 mℓ of 50% methanol/water, followed by adding 42.78 g (0.2 mole) of sodium metaperiodate at 0° C in an argon gas stream and stirring the mixture at room temperature for 4 hours. Then, the reaction solution was filtered off, the filtrate was evaporated to dryness under a reduced pressure, the resulting residue was dissolved in 200 m ℓ of chloroform, and was washed with 10% aqueous solution of sodium bisulfite and then with a saturated aqueous solution of common salt and then dried over anhydrous sodium sulfate. The solvent was evaporated to dryness under a reduced pressure to thus obtain 10.08 g (96.2%) of the title compound (c) as white powder. The product was used in the subsequent reaction without further purification.

Physicochemical Properties of the Compound (c)

TLC (silica gel/chloroform-methanol (10:1)): Rf = 0.44, 0.58
IR $\nu_{max}$ (KBr) $cm^{-1}$ : 3518, 3436, 1745, 1722, 1385, 1321, 1234, 1205, 1089, 1072, 1031.

Reference Example 3: Preparation of Compound (d) [(2R,3S)-1,3-O- benzylidene-4-octadecene-1,2,3-triol]

Triphenyl tetradecyl phosphonium bromide (21.58 g; 40 mmoles) was dissolved in 60 mℓ of anhydrous tetrahydrofuran (THF), 22.4 mℓ (35 mmoles) of butyl lithium (1.56 N) was added to the solution with cooling in an ice-methanol bath (about -20° C) and the mixture was stirred at that temperature for one hour. Then, to this mixture, there was dropwise added a solution of the compound (c) (2.08 g; 10 mmoles) in 15 mℓ of THF at - 20 ° C and stirred for 18 hours at - 20 ° C in room temperature.

The reaction solution was evaporated to dryness under a reduced pressure, the residue obtained was dissolved in 100 m ℓ of chloroform and washed with water and a saturated aqueous solution of common salt, dried over anhydrous magnesium sulfate and then the solvent was distilled off in a reduced pressure. The residue thus obtained was purified by flash chromatography (Wako Gel C-200: 200 g; eluent: hexane/ethyl acetate (9:1)) to give 3.11 g (80.2%) of the title compound (d) as white powder.

Physicochemical Properties of the Compound (d)

TLC (silica gel/hexane-methyl acetate (8:3)): Rf = 0.55
IR $\nu_{max}$ (KBr) $cm^{-1}$ : 3470, 2920, 2840, 1465, 1390, 1115, 1070, 1025.

| Elemental Analysis: (for $C_{25}H_{40}O_3$) | | |
|---|---|---|
| Calc.: | C 77.27; | H 10.37 |
| found: | C 77.13; | H 10.56 |

$^1$H-NMR (500 MHz, $CDCl_3$ + $D_2O$, TMS) $\delta$ (ppm):
0.880 (t, J = 7.0 Hz, $-CH_3$)
1.20~1.42 (m, $-(CH_2)_n-$)
2.10 (q, J = 7.0 Hz, 6-H, H′ trans)
2.10~2.30 (m, 6-H, H′ cis)
3.59~3.72 (m, 1-H, 2-H)
3.94~4.00 (m, 3-H)
4.33~4.40 (m, 1-H′)
5.477 (ddt, J = 11.0, 9.0. 1.5 Hz, 4-H cis)
5.534 (s, 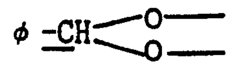 )
5.556 (ddt, J = 15.4, 7.3, 1.5 Hz, 4-H trans)
5.827 (dt, J = 11.0, 7.7Hz, 5-H cis)
5.943 (dt, J = 15.4, 6.9Hz, 5-H trans)

7.33-7.40 (m, phenyl-H)
7.49-7.51 (m, phenyl-H)

The ratio of the 4-trans isomer/the 4-cis isomer of the foregoing compound (d) was determined to be 4.2/1.0 as calculated from the areas of the NMR peak of 5-H.

Reference Example 4: Preparation of the Compound (e) [(2R, 3S)-1,3-O-benzylidene-2-O-methylsulfonyl-4-octadecene-1,2,3-triol]

The compound (d) (1.94 g; 5 mmoles) was dissolved in 5 mℓ of anhydrous pyridine, 0.58 mℓ (7.5 mmoles) of methylsulfonyl chloride was added to the resulting solution with cooling in an ice-methanol bath (about -15 °C). The mixture was stirred for 18 hours at - 15° C in room temperature, then 1 m ℓ of water was added and the mixture was concentrated under a reduced pressure. The resultant residue was dissolved in 50 m ℓ of ether, washed with water and then with a saturated aqueous solution of common salt, dried over anhydrous magnesium sulfate and the solvent was distilled off under a reduced pressure. The residue (2.5 g) thus obtained was purified by flash chromatography (Wako Gel C-300: 110 g; eluent: hexane/ethyl acetate (10:1)) to obtain 2.23 g (95.7%) of the title compound (e) as white powder.

Physicochemical Properties of the Compound (e)

TLC (silica gel/hexane-methyl acetate (4:1)): Rf = 0.34
IR $\nu_{max}$ (KBr) $cm^{-1}$ : 2920, 2840, 1465, 1340, 1180, 1080, 970.

| Elemental Analysis: (for $C_{26}H_{42}O_5S$) | | |
|---|---|---|
| Calc.: | C 66.92; | H 9.07 |
| found: | C 66.86; | H 9.10 |

$^1$H-NMR (500 MHz, $CDCl_3$, TMS) δ (ppm):
0.879 (t, J = 7.0 Hz, $-CH_3$)
1.20~1.41 (m, $-(CH_2)_n-$)
2.084 (q, J = 6.9 Hz, 6-H, H′)
2.995 (s, $CH_3SO_2$-trans)
2.998 (s, $CH_3SO_2$- cis)
3.844 (dd, J = 10.3, 9.8Hz, 1-H trans)
4.194 (dd, J = 9.8, 7.3Hz, 3-H trans)
4.488 (dt, J = 9.8, 9.8 Hz, 2-H trans)
4.536 (dd, J = 10.3, 5.3 Hz, 1-H′ trans)
5.512 (dd, J = 11.0, 8.8 Hz, 4-H cis)

5.546 (s, φ 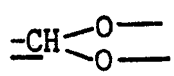 )

5.537 (dd, J = 15.4, 7.3 Hz, 4-H trans)
5.828 (dt, J = 11.0, 7.7Hz, 5-H cis)
5.962 (dt, J = 15.4, 7.0Hz, 5-H trans)
7.35~7.39 (m, phenyl-H)
7.46~7.49 (m, phenyl-H)

The ratio of the 4-trans isomer/the 4-cis isomer of the foregoing compound (d) was determined to be 3.6/1.0 as calculated from the areas of the NMR peak of 5-H.

Reference Example 5: Preparation of the Compound (f) [(2R, 3S)-2-O-methylsulfonyl-4-octadecene-1,2,3-triol]

The compound (e) (13.98 g; 30 mmoles) was dissolved in 300 mℓ of ethanol, and Amberlist-15 (90 g) was added to the solution. The reaction mixture was stirred for 8 hours at 60 - 65 °C, and the resin in the reaction solution was removed by filtration, followed by adding 5 m ℓ of glycerin to the filtrate and then evaporating the solution to dryness. The resulting residue was purified by flash chromatography (Wako Gel C-300: 514 g; eluent: hexane/ethyl acetate (4:1)) to obtain 8.14 g (71.8%) of the title compound (f) as white powder.

Physicochemical Properties of the Compound (f)

TLC (silica gel/ chloroform-methanol (15:1)): Rf = 0.31
IR $\nu_{max}$ (KBr) $cm^{-1}$ : 3304, 2918, 2848, 1466, 1364, 1349, 1175, 930.

| Elemental Analysis: (for $C_{19}H_{38}O_5S$) | | |
|---|---|---|
| Calc.: | C 60.28; | H 10.12 |
| found: | C 66.18; | H 10.15 |

$^1$H-NMR (500 MHz, $CDCl_3/CD_3OD$=9/1, TMS) δ (ppm):
0.882 (t, J = 7.1 Hz, $-CH_3$)
1.2~1.43 (m, $-(CH_2)_n-$)
2.059 (q, J = 7.0 Hz, 6H, H′ trans)
2.06~2.18 (m, 6-H, H′ cis)
3.136(s, $CH_3SO_2$- cis)
2.140 (s, $CH_3SO_2$- trans)
3.779 (dd, J = 12.6, 4.0 Hz, 1-H cis)
3.804 (d, J = 6.2 Hz, 1-H, H′ trans)
3.842 (dd, J = 12.6, 6.6 Hz, 1-H′ cis)
4.329 (dd, J = 7.3, 4.0 Hz, 3-H trans)
4.580 (ddd, J = 6.6, 4.0, 4.4 Hz, 2-H cis)
4.607 (dt, J = 6.2, 4.0 Hz, 2-H trans)
4.711 (ddd, J = 8.8, 4.4, 1.1 Hz, 3-H cis)
5.401 (ddd, J = 11.0, 8.8, 1.5 Hz, 4-H cis)
5.477 (ddd, J = 15.4, 7.3, 1.5 Hz, 4-H trans)
5.658 (ddd, J = 11.0, 7.7, 1.1 Hz, 5-H cis)
5.804 (ddd, J = 15.4, 6.9, 1.1 Hz, 5-H trans)

The ratio of the 4-trans isomer/the 4-cis isomer of the foregoing compound (f) was determined to be 2.7/1.0 as calculated from the areas of the NMR peak of 5-H.

Reference Example 6: Preparation of the Compound (1) [(2R, 3S)-2-O-methylsulfonyl-1,3-O-bis(1-ethoxyethyl)-4-octadecene-1,2,3-triol ]

A solution of compound (f) (2.23 g, 5.9 mmoles), pyridinium p-toluenesulfonate (0.327 g, 1.3 mmoles), and ethyl vinyl ether (9.95 ml, 41.9 mmoles) in $CH_2Cl_2$ (13 ml) was stirred for 1 hour at room temperature. Chloroform (13 ml) was added to the reaction mixture, followed by washing the solution with a saturated aqueous solution of common salt, drying it over anhydrous magnesium sulfate and then distilling off the solvent under a reduced pressure. The residue was purified by flash chromatography (Merck, 120 g of Kieselguhr 60; eluent: hexane/ethyl acetate (4:1)) to obtain 2.93 g (96.8%) of the title compound (1) as colorless oily substance.

Physicochemical Properties of the Compound (1)

TLC (silica gel/ hexane-ethyl acetate (4:1)): Rf = 0.30
IR $\nu_{max}$ (KBr) $cm^{-1}$ : 2920, 2850, 1465, 1360, 1175, 1130, 1085, 1055, 960, 930.

$^1$H-NMR (400 MHz, $CDCl_3$, TMS) δ (ppm):
0.879 (t, J = 7.0 Hz, $-CH_3$)
1.145 ~ 1.227 (m, $CH_3CH_2O$ X 2)
1.279 ~ 1.573 (m, $-(CH_2)_n-$,
O—
$CH_3$- CH -OEt X 2)
2.052 ~ 2.081 (m, 6-H, H′)
3.082 ~ 3.105 (m, $CH_3SO_2$-)
3.432 ~ 3.737 (m, $CH_3CH_2O$- X 2)
4.692 ~ 4.773 (m,
O—
$CH_3$- CH -OEt x 2)
5.30 ~ 5.50 (m, 4-H cis + trans)
5.72 ~ 5.83 (m, 5-H cis + trans)

Example 1: Preparation of (2S, 3S)-2-O-acetyl-1,3-O-bis(1-ethoxyethyl) -4-octadecene-1,2,3-triol (2)

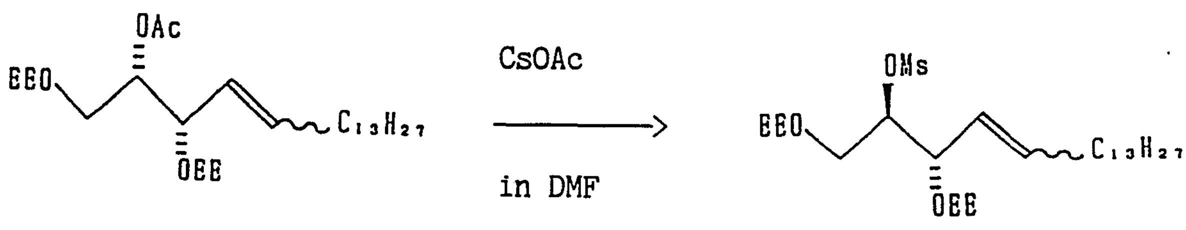


(1) (2)

The compound (1) (103.6 mg; 0.198 mmole) was reacted with 230.3 mg (1.2 mmole) of CsOAc for 91 hours in 2.5 m ℓ of dry DMF at 110 °C. To the reaction solution, there was additionally added 147 mg (1.97 mmole in all) of CsOAc and the mixture was stirred for additional 31 hours at that temperature.

The reaction mixture was diluted 40 mℓ of AcOEt and washed with $NaHCO_3$ and then with 30 m ℓ of a saturated aqueous solution of NaCl, drying over $MgSO_4$ and distilling off AcOEt in a reduced pressure. The residue (85.8 mg) was purified by chromatography (Wako Gel C-300: 4.3 g; eluent: hexane/AcOEt (19:1)) to obtain 67.0 mg (69.5%) of the title compound (2) and 10.7 mg of the starting material (compound (1)). The yield of the compound (2) was 77.5% if the amount of the starting material recovered was taken into consideration.

Physicochemical Properties of the Compound (2)

TLC (silica gel; hexane-AcOEt (4:1)): Rf = 0.31
IR $\nu_{max}$ (neat) $cm^{-1}$: 2922, 2850, 1746, 1373, 1236, 1136, 1086, 1055.

| Elemental Analysis (for $C_{28}H_{54}O_6$) | | |
|---|---|---|
| Calcd. : | C 69.10; | H 11.18 |
| Found : | C 68.98; | H 11.07 |

$^1$H-NMR (500 MHz, $CDCl_3$, TMS, 24 °C) δ (ppm):
0.880 (3H, t, J = 7.0 Hz, $-CH_3$)
1.190 (6H, t, J = 7.1 Hz, $OCH_2CH_3$ X 2)

1.24~1.40 (28H, m, $CH_2$ X 11 and O -CHCH$_3$ X 2)
O

2.02~2.06 (2H, m, 6-$CH_2$)
2.075 (3H, S, $OCOCH_3$)
5.68 ~5.74 (1H, m, H-5 (cis + trans))

Example 2: Preparation of (2S, 3S)-1,3-O-bis(1-ethoxyethyl)-4-octadecene-1,2,3-triol (3)

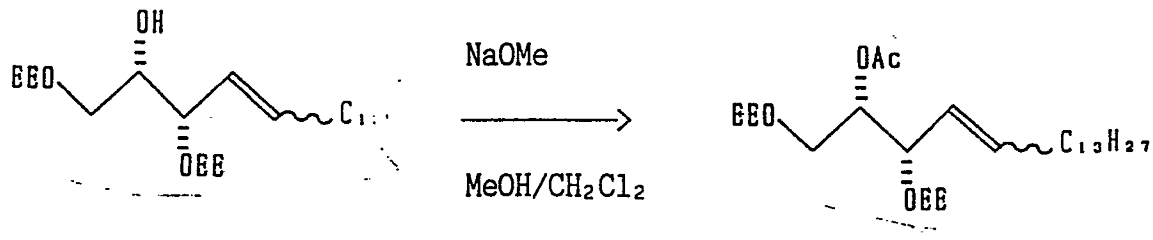


(2) (3)

The compound (2) (1.9988 mg; 4.1 mmoles) was dissolved in a mixture of 20 mℓ of MeOH and 20 mℓ of $CH_2Cl_2$ and 4.1 mℓ (4.1 mmoles) of 1N NaOMe solution in MeOH and a mixture was stirred for is hours at room temperature. The reaction solution was neutralized with Amberlist-15 ($H^+$ type) and evaporated to dryness under a reduced pressure to obtain 1.6773 g (91.1%) of pale yellow oily substance. A part of the oily substance was purified by chromatography (Wako Gel C-300; eluent: hexane/AcOEt (9:1)) to separate into two fractions having Rf values of 0.45 and 0.39 respectively. These two products are stereoisomers with respect to the ethoxyethyl group. IR measurement and elemental analysis were performed on these two products. The remaining product was used in the subsequent process without further purification.

Physicochemical Properties of the Compound (3)

TLC (silica gel; hexane-AcOEt (2:1)): Rf = 0.45, 0.39, 0.34
IR $\nu_{max}$ (neat) $cm^{-1}$ : 3466, 2920, 2850, 1464, 1378, 1134, 1091, 1056.

| Elemental Analysis (for $C_{26}H_{52}O_5 \cdot 1/4H_2O$) | | |
|---|---|---|
| Calc. : | C 69.52; | H 11.78 |
| Found : | C 69.60; | H 11.66 |

$^1$H-NMR (500 MHz, $CDCl_3$, TMS, 24 °C) δ (ppm) for the product having Rf of 0.45:
0.881 (3H, t, J = 7.0 Hz, -$CH_3$)
1.198 (6H, t, J = 7.1 Hz, $OCH_2CH_3$ X 2)
1.22~1.42 (28H, m, $CH_2$ X 11 and O- CHCH$_3$ X 2)
O
2.055 (2H, q, J = 6.8 Hz, 6-$CH_2$)
2.848 (0.5H, d, J = 1.8 Hz, OH)
2.911 (0.5H, d, J = 3.3 Hz, OH)
5.30 ~5.42 (1H, m, 4-H (cis + trans))
5.61 ~5.76 (1H, m, 5-H (cis + trans))
$^1$H-NMR (500 MHz, $CDCl_3$, TMS, 24 °C) δ (ppm) for the product having Rf of 0.39:
0.881 (3H, t, J = 7.0 Hz, -$CH_3$)
1.173 (3H, t, J = 7.0 Hz, $OCH_2CH_3$)

1.197 (3H, t, J = 7.1 Hz, $OCH_2CH_3$)
1.22~1.41 (28H, m, $CH_2$ X 11 and O-$\underset{O}{C}HCH_3$ X 2)
2.056 (2H,q, J = 6.7 Hz, 6-$CH_2$)
2.713 (0.5H, d, J = 3.3 Hz, OH)
2.768(0.5H, d, J = 3.7 Hz, OH)
3.85 ~3.89 (1H, m, H-3)
5.442 (1H, dd, J = 8.4 and 15.4 Hz, H-4 (trans))
5.718 (1H, m, H-5 (trans))

Example 3: Preparation of (2S, 3S)-1,3-O-bis(1-ethoxyethyl)-2-O-methylsulfonyl-4-octadecene-1,2,3-triol (4)

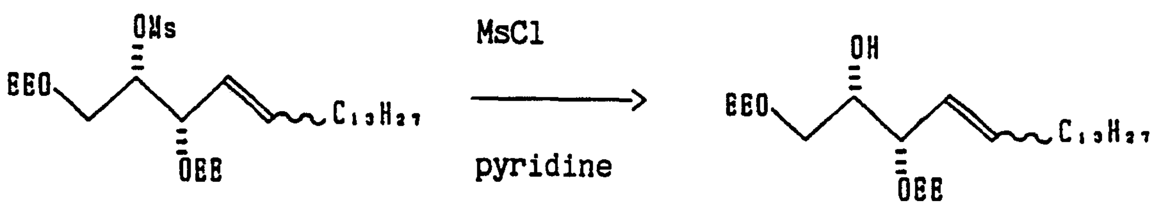


(3) (4)

The compound (3) (crude product; 1.6500 g (3.7 mmoles) was dissolved in 41 mℓ of dry pyridine, and then 433μ ℓ (5.6 mmoles) of MsCl was added to the solution. The mixture was stirred for 20 hours at room temperature, and the reaction solution was concentrated under a reduced pressure. The residue (1.91 g) was purified by chromatography (Wako Gel C-300: 95.5 g; eluent: hexane/AcOEt (7:1)) to obtain 1.4370 g (74.0%) of the title compound (4). At this stage, the fractions having Rf values of 0.46 and 0.43 respectively were isolated for NMR measurement. These two products are stereoisomers with respect to their ethoxyethyl group.

Physicochemical Properties of the Compound (4)

TLC (silica gel; hexane-AcOEt (2:1)): Rf = 0.46, 0.43
IR $\nu_{max}$ (neat) $cm^{-1}$ : 2922, 2850, 1457, 1360, 1177, 1136, 1086, 1056, 929.

| Elemental Analysis (for $C_{27}H_{54}O_7S$) | | |
|---|---|---|
| Calc. : | C 62.03; | H 10.41 |
| Found : | C 62.32; | H 10.03 |

$^1$H-NMR (500 MHz, $CDCl_3$, TMS, 24 °C) δ (ppm) for the product having Rf of 0.46:
0.881 (3H, t, J = 7.5 Hz, $-CH_3$)
1.18~1.21 (6H, m, $OCH_2CH_3$ X 2)
1.22~1.42 (28H, m, $CH_2$ X 11 and
O-$\overset{O}{C}HCH_3$ X 2)
2.066 (2H,q, J = 6.7 Hz, 6-$CH_2$)
3.093, 3.101, 3.104, 3.111 (4S, $CH_3SO_2$)
$^1$H-NMR (500 MHz, $CDCl_3$, TMS, 24 °C) δ (ppm) for the product having Rf of 0.43:
0.880 (3H, t, J = 7.0 Hz, $-CH_3$)

1.15~1.21 (6H, m, $OCH_2CH_3$ X 2)
1.22~1.41 (28H, m, $CH_2$ X 11 and O-$CHCH_3$ X 2)
O

2.059 (2H,q, J = 6.6 Hz, 6-$CH_2$)
3.077, 3.085 (2S, $CH_3SO_2$)

Example 4: Preparation of (2R, 3S)-2-azido-1,3-O-bis(1-ethoxyethyl)- 4-octadecene-1,3-diol (5)

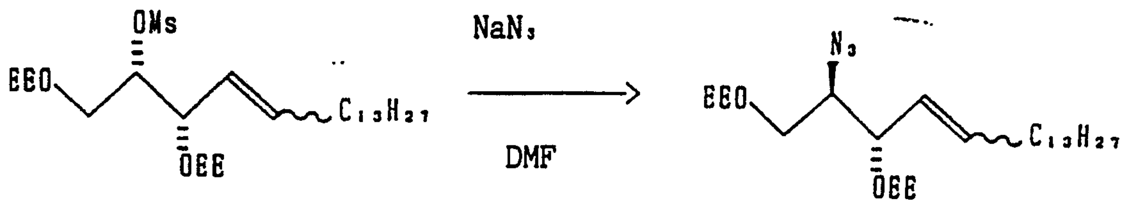


(4) (5)

The compound (4) (1.4000 g; 2.7 mmoles) was dissolved in 28 mℓ of dry DMF, and then 1.0455 g (16.1 mmoles) of $NaN_3$ was added to the solution to carry out the reaction at 100 ° C for 43 hours in an oil bath. The reaction solution was filtered, and a filtrate was evaporated to dryness under a reduced pressure. The residue obtained (1.3220 g) was purified by chromatography (Wako Gel C-300: 66.1 g; eluent: hexane/AcOEt (9:1)) to obtain 1.0092 g (80.2%) of the title compound (5).

Physicochemical Properties of the Compound (5)

TLC (silica gel; hexane-AcOEt (6:1)): Rf = 0.48, 0.43, 0.38
IR $\nu_{max}$ (neat) $cm^{-1}$ : 2922, 2852, 2096, 1458, 1378, 1135, 1087, 1057.

| Elemental Analysis (for $C_{26}H_{51}N_3O_4$) | | | |
|---|---|---|---|
| Calc. : | C 66.49; | H 10.94; | N 8.95 |
| Found : | C 66.94; | H 11.06; | N 8.39 |

$^1$H-NMR (500 MHz, $CDCl_3$, TMS, 24 ° C) δ (ppm):
0.880 (3H, t, J = 7.5 Hz, -$CH_3$)
1.16~1.22 (6H, m, $OCH_2CH_3$ X 2)
1.22~1.34 (26H, m, $CH_2$ X 10 and O-$CHCH_3$)
O
1.34~1.42 (2H, m, 7-$CH_2$)
2.03~2.12 (2H. m, 6-$CH_2$)

Example 5: Preparation of (2R, 3S 4E)-2-azido-4-octadecene-1,3-diol (6) and (2R, 3S, 4Z)-2-azido-4-octadecene-1,3-diol (7)

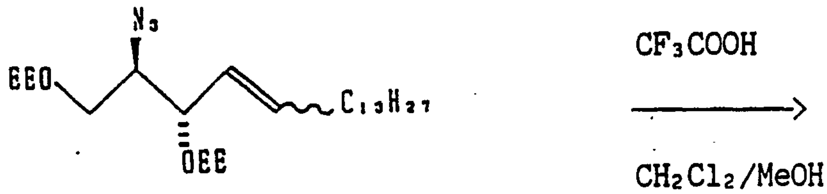


(5)

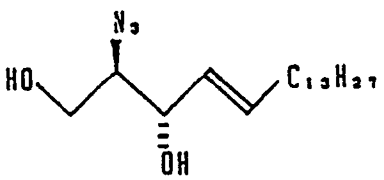

+

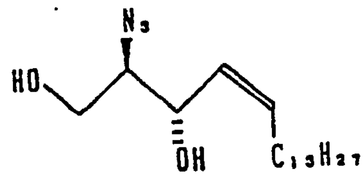

(6) (7)

The compound (5) (0.9978 g; 2.12 mmoles) was dissolved in a mixture of 10 mℓ of $CH_2Cl_2$ and 10 mℓ of MeOH, 327 μ ℓ (4.25 mmoles) of $CF_3COOH$ was added to the resulting solution with cooling it in an icebath, the mixture was reacted at that temperature for 2 hours and the reaction was further continued for additional is hours at room temperature. The reaction solution was evaporated to dryness under a reduced pressure, the resulting residue (0.7798 g) was purified by chromatography (Wako Gel C-300: 39 g; eluent: hexane/AcOEt (3/1)) to thus obtain 78.6 mg of the compound (6), 7.2 mg of the compound (7) and 554.2 mg of a mixture of these compounds (total yield of the compounds (6) and (7): 92.6%). The mixture of the compounds (6) and (7) (554.2 mg) thus obtained was subjected to fractional recrystallization from hexane to thus obtain 349.6 mg of the compound (6) as colorless needles. The resulting recrystallization mother liquor was evaporated to dryness under a reduced pressure and the residue obtained was purified by chromatography (Wako Gel C-300: 07.2 g; eluent: hexane/AcOEt (4/1)) to thus obtain 69.5 mg of the compound (6) (total yield of the compound (6): 72.0%), 47.4 mg of the compound (7) (total yield of the compound (7): 7.9%) and 42.2 mg of a mixture of these compounds (6) and (7).

The compound (6) was recrystallized from n-pentane to obtain colorless needles. On the other hand, the compound (7) was recrystallized from n-pentane to obtain colorless needles.

Physicochemical Properties of the Compound (6)

TLC: (silica gel; hexane/AcOEt (1:1)): Rf = 0.34
m.p.: 53 - 54 ° C
[ α ] $_D$ (21 ° C): + 34.0 ° C (C = 1.00; in $CHCl_3$)
IR$\nu_{max}$ (KBr) $cm^{-1}$ : 3314, 2916, 2846, 2142, 1465, 1362, 1275, 1000, 965.

| Elemental Analysis (for $C_{18}H_{35}N_3O_2$) | | | |
|---|---|---|---|
| Calc. : | C 66.42; | H 10.84; | N 12.91 |
| Found : | C 66.72; | H 10.79; | N 13.13 |

$^1$H-NMR (500 MHz, CDCℓ $_3$, TMS, 24 ° C) δ (ppm):

0.881 (3H, t, J = 7.0 Hz, $CH_3$)
1.20~1.35 (20H, m, $CH_2$ X 10)
1.35-1.43 (2H, m, 7-$CH_2$)
2.073 (2H,q, J = 7.0 Hz, 6-$CH_2$)
3.509(1H, m, 2-H)
3.768 (1H, dd, J = 11.7, 5.9 Hz, 1-H)
3.804 (1H, dd, J = 11.7, 4.8 Hz, 1′-H)
4.252 (1H, t, J = 6.6 Hz, 3-H)
5.540 (1H, ddt, J = 15.3, 7.3, 1.5 Hz, 4-H)
5.822 (1H, dtd, J = 15.3, 6.8, 0.9 Hz, 5-H)

Physicochemical Properties of the Compound (7)

TLC: (silica gel; hexane/AcOEt (1:1)): Rf = 0.40
m.p.: 43 - 44.5 ° C
[ a ] D (22 ° C): + 52.3 ° (C = 1.01; in $CHCl_3$)
IR$\nu_{max}$ (KBr) $cm^{-1}$ : 3290, 2918, 2134, 1465, 1354, 1278, 1004.

| Elemental Analysis (for $C_{18}H_{35}N_3O_2$) | | | |
|---|---|---|---|
| Calc. : | C 66.42; | H 10.84; | N 12.91 |
| Found : | C 66.37; | H 10.79; | N 12.89 |

$^1$H-NMR (500 MHz, $CDCl_3$, TMS, 24° C) δ (ppm):
0.881 (3H, t, J = 7.0 Hz, $CH_3$)
1.18~1.34 (20H, m, $CH_2$ X 10)
1.34~1.43 (2H, m, 7-$CH_2$)
1.850 (1H, d, J = 4.0 Hz, 3-OH)
2.000 (1H, t, J = 6.0 Hz, 1-OH)
2.05~2.16 (2H, m, 6-$CH_2$)
3.528 (1H, dd, J = 5.9, 10.6 Hz, H-2)
3.76~3.84 (2H, m, H-1 and H′-1)
4.609 (1H, dt, J = 4.8, 9.3 Hz, H-3)
5.484 (1H, dd, J = 9.0, 11.3 Hz, H-4)
5.695 (1H, dt, J = 7.5, 11.0 Hz, H-5)

Example 6: Preparation of (2R, 3S, 4E)-2-azido-1,3-O-bis(1-ethoxyethyl)-4-octadecene-1,3-diol (8)

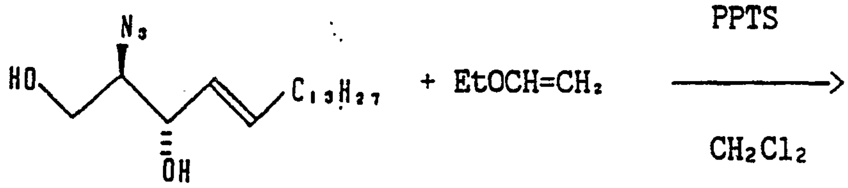


(6)

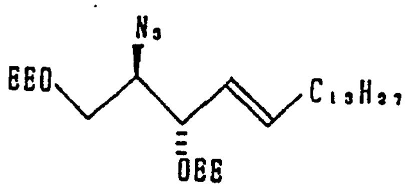

(7)

The compound (6) (378.0 mg; 1.16 mmole) was dissolved in 22 mℓ of anhydrous dichloromethane and 0.8 mℓ of ethyl vinyl ether and 37. 9 mg (0.15 mmole) of pyridinium p-toluenesulfonate were added to the resulting solution to perform the reaction at room temperature for 1.5 hour. The reaction solution was evaporated to dryness under a reduced pressure and the residue obtained was purified by silica gel column chromatography (Wako Gel C-300: 21 g; eluent: hexane/AcOEt (9/1)) to thus obtain 540.7 mg (99.1%) of the title compound (8). The product thus obtained was used in the subsequent process without complete purification.

TLC: (silica gel; hexane/AcOEt (6:1)): Rf = 0.48, 0.43

Example 7: Preparation of (2R, 3S, 4Z)-2-azido-1,3-O-bis(1-ethoxyethyl)-4-octadecene-1,3-diol (9)

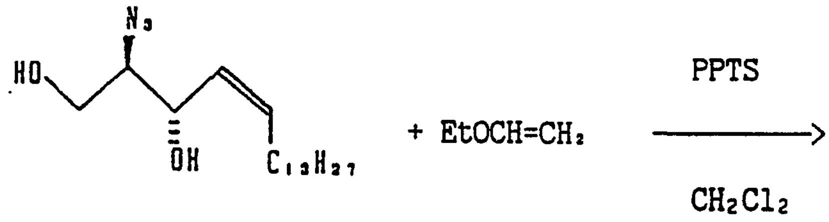


(7)

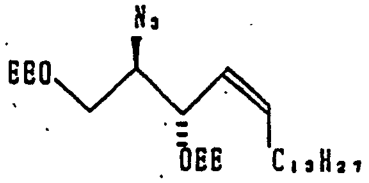

(9)

The compound (7) (49.9 mg; 0.153 mmole) was dissolved in 3 mℓ of anhydrous dichloromethane and 0.1 mℓ of ethyl vinyl ether and 5 mg (0.02 mmole) of pyridinium p-toluenesulfonate were added to the resulting solution to perform the reaction at room temperature for 3 hours. The reaction solution was evaporated to dryness under a reduced pressure and the residue obtained was purified by silica gel column chromatography (Wako Gel C-300: 4 g; eluent: hexane/AcOEt (9/1)) to thus obtain 57.0 mg (79.2%) of the title compound (9). The product thus obtained was used in the subsequent process without complete purification.

TLC: (silica gel; hexane/AcOEt (6:1)): Rf = 0.51, 0.44

Example 8: Preparation of (2R, 3S, 4E)-2-amino-1,3-O-bis(1-ethoxyethyl)-4-octadecene-1,3-diol (10)

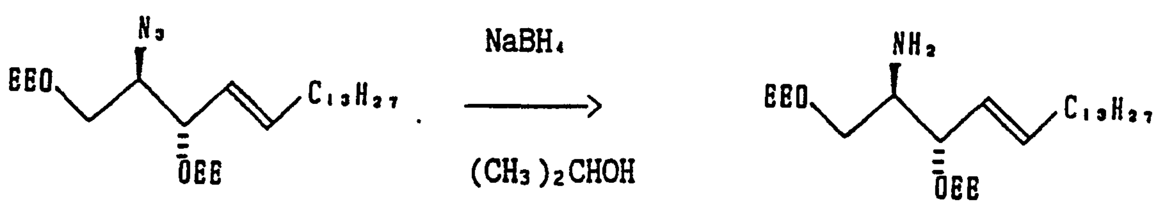


(8) (10)

A mixture of compound (8) (540.7 mg, 1.15 mmoles) and $NaBH_4$ (348. 4 mg, 9.21 mmoles in isopropanol (20 ml) was stirred for 42 hours under reflux. Chloroform (100 m ℓ ) was added to the reaction solution, insoluble matters formed at this time were removed by filtration and the filtrate was evaporated to dryness under a reduced pressure. The residue obtained was purified by silica gel column chromatography (Wako Gel C-300: 25.5 g; eluent: $CHCl_3$/MeOH (19/1)) to thus obtain 458. 0 mg (89.7%) of the title compound (10).

Physicochemical Properties of the Compound (10)

TLC: (silica gel; $CHCl_3$/MeOH (10:1)): Rf = 0.50
$IR\nu_{max}$ (neat) $cm^{-1}$ : 2972, 2922, 2852, 1457, 1378, 1133, 1090, 1057.

| Elemental Analysis (for $C_{26}H_{53}NO_4$) | | | |
|---|---|---|---|
| Calc. : | C 70.38; | H 12.04; | N 3.16 |
| Found : | C 70.23; | H 11.81; | N 2.99 |

$^1$H-NMR (500 MHz, $CDCl_3$, TMS, 24° C) δ (ppm):
0.880 (3H, t, J = 7.0 Hz, $CH_3$)
1.15~1.41 (34H, m, $CH_2$ X 11, $\underline{CH_3}CH_2O$ X 2 and
O
|
O-CHCH$_3$ X 2)
2.073 (2H,q, J = 7.1 Hz, 6-$CH_2$)
5.326 (0.5H, ddt, J = 1.5, 8.6, 15.6 Hz, H-4)
5.438 (0.5H, ddd, J = 2.2, 8.4, 15.4 Hz, H4)
5.66~5.73 (1H, m. H-5)

Example 9: Preparation of (2R, 3S, 4z)-2-amino-1,3-O-bis(1-ethoxyethyl)-4-octadecene-1,3-diol (11)

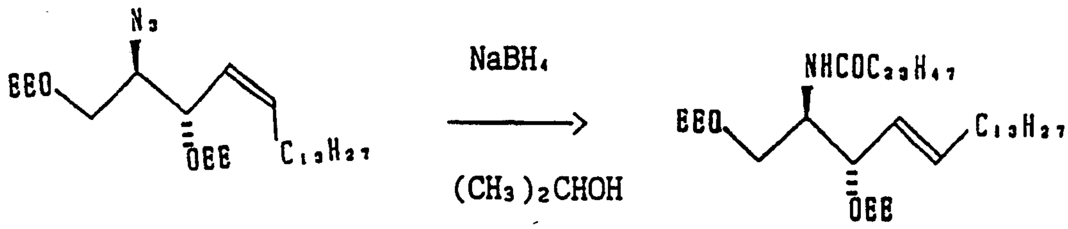


(9) (11)

A mixture of compound (9) (57.0 mg, 0.12 mmole) and $NaBH_4$ (36.7 mg, 0.97 mmole) in iso-propanol (3 ml) was stirred for 40 hours under reflux. 30% AcOH/MeOH was added to the reaction solution to decompose excess sodium borohydride and the reaction solution was evaporated to dryness under a reduced pressure. A solution of residue in $CHCl_3$ (60 ml) was washed with water and saturated aqueous Nacl, dried over anhydrous magnesium sulfate and then the chloroform was removed by distillation under a reduced pressure. The resulting residue was purified by silica gel column chromatography (Wako Gel C-300: 5.2 g; eluent: $CHCl_3$/MeOHt (19/1)) to thus obtain 43.2 mg (80.3%) of the title compound (11).

Physicochemical Properties of the Compound (11)

TLC: (silica gel; $CHCl_3$/MeOHt (12:1)): Rf = 0.53
$^1$H-NMR (500 MHz, $CDCl_3$, TMS, 24° C) δ (ppm):
0.880 (3H, t, J = 7.0 Hz, $CH_3$)
1.16~1.41 (34H, m, $CH_2$ X 11, $\underline{CH_3}CH_2O$ X 2 and O-CH$\underline{CH_3}$ X 2)
O
2.03~2.17 (2H, m, 6-$CH_2$)
5.274 (0.5H, t, J = 10.4 Hz, H-4)
5.401 (0.5H, dt, J = 1.5, 10.3 Hz, H-4)
5.64~5.75 (1H, m, H-5)

Example 10: Preparation of (2R, 3S, 4E)-2-tetracosamido-1,3-O-bis(1-ethoxyethyl)-4-octadecene-1,3-diol (12)

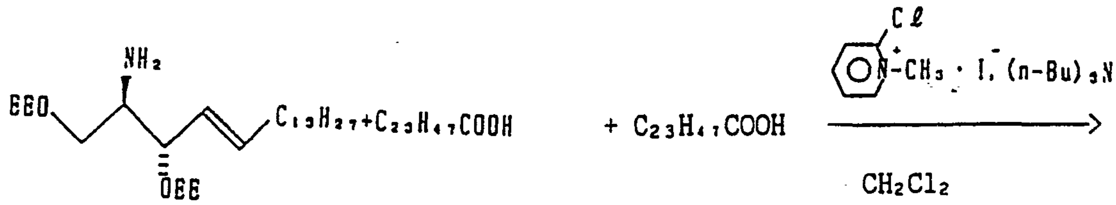


(10)

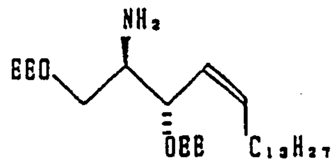

(12)

The compound (10) (440.0 mg; 0.992 mmole), lignoceric acid (438.7 mg; 1.19 mmole), 2-chloro-1-methylpyridinium iodide (380.7 mg; 1.49 mmole) and tri-n-butylamine (0.945 mℓ; 3.97 mmoles) were refluxed for one hour in 20 mℓ of anhydrous dichloromethane. The reaction solution was evaporated to dryness under a reduced pressure and the resulting residue was dissolved in 250 m ℓ of ether, the solution was washed with 150 m ℓ each of water three times and further with 150 mℓ each of a saturated aqueous solution of common salt three times, dried over anhydrous magnesium sulfate and ether was distilled off under a reduced pressure to thus obtain 783.6 mg (99.5%) of the compound (12) as white powder. A part thereof was purified and separated into the products having Rf values of 0.44 and 0.37 respectively and the NMR measurement was performed on these products. These two compounds are stereoisomers with respect to the ethoxyethyl group.

Physicochemical Properties of the Compound (12)

TLC: (silica gel; hexane/AcOEt (7:3)): Rf = 0.44, 0.37
$IR\nu_{max}$ (KBr) $cm^{-1}$: 3318, 2914, 2846, 1641, 1543, 1465, 1377, 1135, 1060.

| Elemental Analysis (for $C_{50}H_{99}NO_5$) | | | |
|---|---|---|---|
| Calc. : | C 75.60; | H 12.56; | N 1.76 |
| Found : | C 75.88; | H 12.59; | N 1.61 |

$^1$H-NMR (500 MHz, $CDCℓ_3$, TMS, 24° C) δ (ppm) for the product having Rf of 0.44:
0.880 (6H, t, J = 7.0 Hz, $CH_3$ X 2)
1.10~1.43 (74H, m, $CH_2$ X 31, $OCH_2CH_3$ X 2 and O-CHCH3 X 2)

2.026 (2H,q, J = 7.0 Hz, 6-$CH_2$)
2.126 (2H, m, $NHCO\underline{CH_2}$)
5.326 (1H, m, H-4)
5.651 (1H, m, H-5)
5.840 (0.5H, d, J = 6.5 Hz, NH)
5.857 (0.5H, d, J = 7.2 Hz, NH)
$^1$H-NMR (500 MHz, $CDCl_3$, TMS, 24° C) δ (ppm) for the product having Rf of 0.37:
0.880 (6H, t, J = 7.0 Hz, $CH_3$ X 2)
1.17~1.39 (74H, m, $CH_2$ X 31, $OCH_2\underline{CH_3}$ X 2 and $O\text{-}\underset{O}{C}H\underline{CH_3}$ X 2)
2.028 (2H,q, J = 7.1 Hz, 6-$CH_2$)
2.116 (2H, m, $NHCO\underline{CH_2}$)
5.428 (1H, m, H-4)
5.62~5.74 (2H, m, H-5 and NH)

Example II: Preparation of (2R, 3S, 4Z)-2-tetracosamido-1,3-O-bis(1-ethoxyethyl)-4-octadecene-1,3-diol (13)

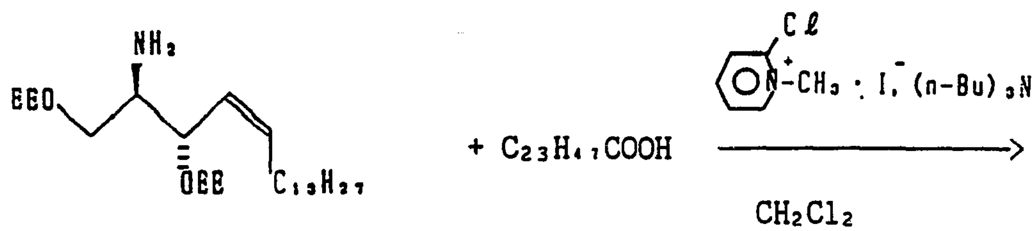


(11)

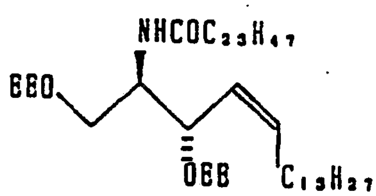

(13)

The compound (11) (36.6 mg; 82 μ moles), lignoceric acid (36.5 mg; 99μ moles), 2-chloro-1-methylpyridinium iodide (31.6 mg; 124μ moles) and tri-n-butylamine (79 μ ℓ ; 330 μ moles) were refluxed for two hours in 2 mℓ of anhydrous dichloromethane on an oil bath maintained at 70 ° C. After the reaction, the reaction solution was evaporated to dryness under a reduced pressure. The resulting residue was dissolved in 100 mℓ of ether, the solution was washed with 50 m ℓ each of water four times and further with 50 mℓ each of a saturated aqueous solution of common salt two times, dried over anhydrous magnesium sulfate and ether was distilled off under a reduced pressure to thus obtain 79.0 mg of white powder. The white powder was purified by silica gel column chromaography (Wako Gel C-300: 4 g; eluent: hexane/AcOEt (9:1)) to obtain 64.2 mg (98.0%) of the title compound (13).

Physicochemical Properties of the Compound (13)

TLC: (silica gel; hexane/AcOEt (7:3)): Rf = 0.46, 0.43
$IR_{\nu\,max}$ (KBr) $cm^{-1}$: 3308, 2914, 2846, 1647, 1551, 1466, 1383, 1133.

| Elemental Analysis (for $C_{50}H_{99}NO_5$) | | | |
|---|---|---|---|
| Calc. : | C 75.60; | H 12.56; | N 1.76 |
| Found : | C 75.81; | H 12.43; | N 1.76 |

$^1$H-NMR (500 MHz, $CDCl_3$, TMS, 24° C) δ (ppm):
0.880 (6H, t, J = 7.0 Hz, $CH_3$ X 2)
1.11~1.41 (72H, m, $CH_2$ X 30, $OCH_2\underline{CH_3}$ X 2 and O-$\underset{O}{C}$H$\underline{CH_3}$ X 2)
1.55~1.64 (2H, m, NHCOCH$_2$ $CH_2$)
2.20~2.19 (4H, m, $NHCO\underline{CH_2}\overline{CH_2}$ and 6-$CH_2$)

Example 12: Preparation of (2R, 3S, 4E)-N-tetracosanoyl sphingenine (14)

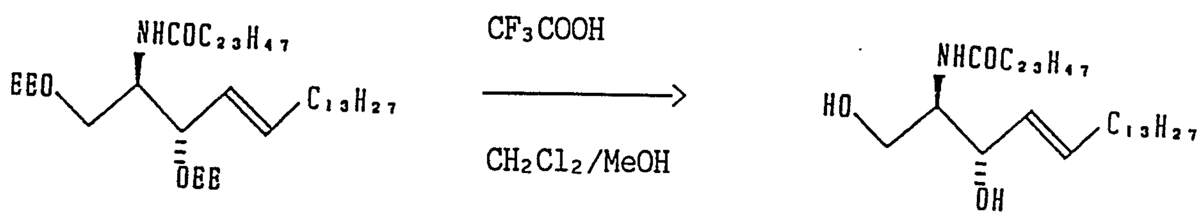


(12)

(14)

The compound (12) (351.3 mg; 0.44 mmole) was dissolved in 1:1 $CH_2Cl_2$-MeOH (14 ml) and 68 μl - (0.88 mmole) of $CF_3COOH$ was added to the resulting solution to perform the reaction at room temperature for 3.5 hours. The reaction solution was evaporated to dryness under a reduced pressure and the residue was recrystallized from acetone to thus obtain 238.9 mg (83.1%) of the title compound (14) as colorless prismatic crystals.

Physicochemical Properties of the Compound (14)

TLC: (silica gel; $CHCl_3$/MeOH (15:1)): Rf = 0.36
$[\alpha]_D$ (22 ° C): + 7.94 ° (C = 1.03; in pyridine)
m.p.: 97.5 - 99 ° C
$IR_{\nu\,max}$ (KBr) $cm^{-1}$: 3374, 2916, 2846, 1646, 1630, 1549, 1470, 1045.

| Elemental Analysis (for $C_{42}H_{83}NO_3$) | | | |
|---|---|---|---|
| Calc. : | C 77.59; | H 12.87; | N 2.15 |
| Found : | C 77.47; | H 12.84; | N 2.04 |

$^1$H-NMR (500 MHz, $CDCl_3$/$CD_3OD$ = 9/1, TMS, 24° C) δ (ppm):
0.883 (6H, t, J = 7.0 Hz, $CH_3$ X 2)
1.17~1.42 (62H, m, $CH_2$ X 31)
1.610 (2H, m, $NHCOCH_2\underline{CH_2}$)

2.041 (2H,q, J = 7.1 Hz, 6-$CH_2$)
2.208 (2H, t, J = 7.5 Hz, $NHCOCH_2$)
3.642 (1H, dd, J = 5.3 and 13.0 Hz, H-1)
3.81~3.84 (2H, m, H-1′ and H-2)
4.163 (1H, t, J = 5.5 Hz, H-2)
5.479 (1H, dd, J = 6.6 and 15.4 Hz, H-4)
5.740 (1H, dt, J = 7.2 and 15.5 Hz, H-5)

Example 13: Preparation of (2R, 3S, 4Z)-N-tetracosanoyl sphingenine (15)

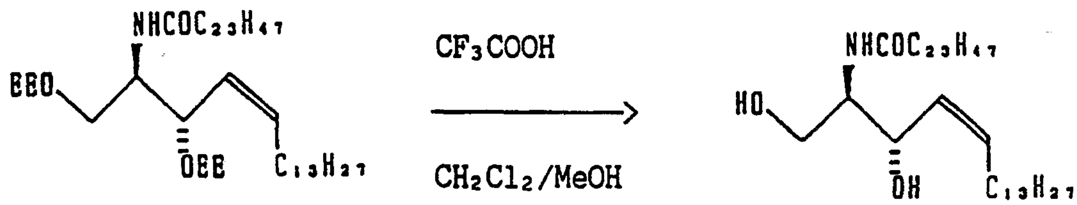


(13) (15)

The compound (13) (50.4 mg; 63.4 μ moles) was dissolved in 1:1 $CH_2Cl_2$-MeOH (2 ml), and 9.8 in μ ℓ (126.9 μ moles) of $CF_3COOH$ was added to the resulting solution to perform the reaction at room temperature for 4 hours. The reaction solution was evaporated to dryness under a reduced pressure and the residue was recrystallized from acetone to thus obtain 31.6 mg (76.7%) of the title compound (15) as colorless needles.

Physicochemical Properties of the Compound (15)

TLC: (silica gel; $CHCl_3$/MeOH (15:1)): Rf = 0.38
$[\alpha]_D$ (24 °C): + 8.57 ° (C = 0.51; in pyridine)
m.p.: 90 - 91.5 ° C
IR$\nu_{max}$ (KBr) $cm^{-1}$: 3302, 2914, 2846, 1647, 1540, 1468, 1260, 1026.

| Elemental Analysis (for $C_{42}H_{83}NO_3$ •1/4 $H_2O$) | | | |
|---|---|---|---|
| Calc. : | C 77.06; | H 12.86; | N 2.14 |
| Found : | C 76.95; | H 12.76; | N 1.99 |

$^1$H-NMR (500 MHz, CDCℓ$_3$/$CD_3OD$ = 9/1, TMS, 24° C) δ (ppm):
0.882 (6H, t, J = 7.0 Hz, $CH_3$ X 2)
1.18~1.43 (62H, m, $CH_2$ X 31)
1.614 (2H, m, $NHCOCH_2CH_2$)
2.01~2.15 (2H, m, 6-$CH_2$)
2.206 (2H, t, J = 7.7 Hz, $NHCOCH_2$)
3.658 (1H, dd, J = 3.5 and 11.2 Hz, H-1)
3.811 (1H, dt, J = 3.9 and 5.5 Hz, H-2)
3.864 (1H, dd, J = 4.4 and 11.4 Hz, H-1′)
4.526 (1H, dd, J = 5.3 and 7.9 Hz, H-3)
5.435 (1H, dd, J = 9.0 and 10.8 Hz, H-4)
5.572 (1H, dt, J = 7.0 and 11.0 Hz, H-5)

## Claims

1. A sphingenine and derivatives thereof represented by the following general formulas (I) and (II):

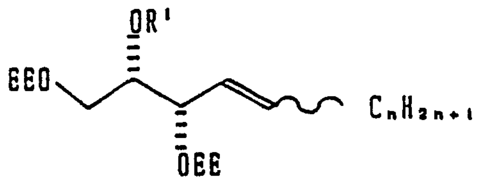

(I)

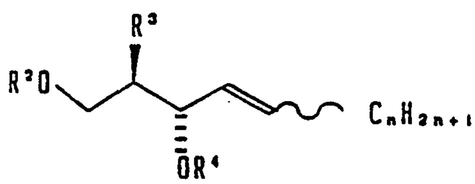

(II)

in the formulas (I) and (II), $R^1$ represents a hydrogen atom, an acetyl group or a methanesulfonyl group; $R^2$ represents a hydrogen atom or an ethoxyethyl group; $R^3$ represents an azido group, an amino group, an alkylamido group, an arylamido group, an aralkylamido group or a tetracosanamido group; $R^4$ represents a hydrogen atom or an ethoxyethyl group; EE represents an ethoxy ethyl group; and n is an integer from 1 to 22.

2. A method for preparing a compound of the general formulas (I) and (II) comprising reacting a compound of the general formula

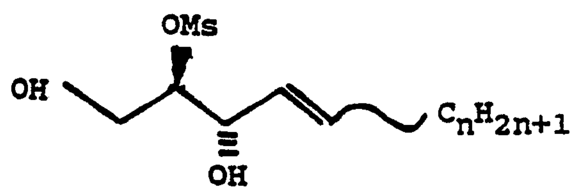

wherein Ms is a methylsulfonyl group with ethyl vinyl ether and optionally converting the obtained compound of the formula (I) wherein $R'$ is a methylsulfonyl group in a per se known manner into the derivatives of the formula (II) or into a compound of the formula (I) wherein $R'$ is a hydrogen atom or an acetyl group.

3. The use of the compounds of the general formulas (I) or (II) according to claim 1 for the preparation of glycolipids and gangliosides.